# EUROPEAN PATENT SPECIFICATION

(11) **EP 3 238 707 B1**
(45) Date of publication and mention of the grant of the patent: **17.02.2021**
(21) Application number: 17176231.3
(22) Date of filing: 21.10.2014
(51) Int. Cl.: A61K 9/00, A61K 45/06, A61K 47/10, A61K 47/12, A61K 47/22, A61K 31/436, A61K 31/19, A61K 31/198, A61K 31/20, A61K 31/216, A61K 31/37, A61K 31/4166, A61K 31/55, A61K 38/34, A61P 3/10

(54) **COMPOSITIONS AND METHODS FOR TREATING INTESTINAL HYPERPERMEABILITY**
ZUSAMMENSETZUNGEN UND VERFAHREN ZUR BEHANDLUNG INTESTINALER HYPERPERMEABILITÄT
COMPOSITIONS ET PROCÉDÉS DE TRAITEMENT DE L'HYPERPERMÉABILITÉ INTESTINALE

(30) Priority: 22.10.2013 US 201361894261 P; 24.10.2013 US 201314062165
(43) Date of publication of application: 01.11.2017
(62) Divisional of application: 14793397.2
(73) Proprietor: Hoffman, Steve, Mahwah, NJ 07430 (US)
(72) Inventor: Hoffman, Steve, Mahwah, NJ 07430 (US)
(74) Representative: Carpmaels & Ransford LLP

(56) References cited:
- YAMADA JUN ET AL: "Involvement of adrenaline in diazepam-induced hyperglycemia in mice", LIFE SCIENCES, PERGAMON PRESS, OXFORD, GB, vol. 66, no. 13, 18 February 2000 (2000-02-18), pages 1213-1221, XP008185720, ISSN: 0024-3205, DOI: 10.1016/S0024-3205(00)00426-4 [retrieved on 2000-03-13]
- FUJIMORI K ET AL: "Mechanisms of hyperglycemic response to chlorpromazine administered into lateral ventricle in rats-I: Possible role of dopaminergic nervous system", NEUROPHARMACOLOGY, PERGAMON PRESS, OXFORD, GB, vol. 13, no. 4, 1 April 1974 (1974-04-01), pages 245-254, XP023822441, ISSN: 0028-3908, DOI: 10.1016/0028-3908(74)90074-4 [retrieved on 1974-04-01]

## Description

### TECHNICAL FIELD

The present invention relates generally to treatment regimens, compositions, and kits for the treatment of hyperglycemia, as defined in the claims.

### BACKGROUND

Yamada Jun et al., Life Sciences, Pergamon Press, Oxford, GB, vol. 66, no. 13, pages 1213-1221, concerns: "Involvement of adrenaline in diazepam-induced hyperglycemia in mice". Fujimori K et al., Neuropharmacology, Pergamon Press, Oxford, GB, vol. 13, no. 4, concerns: "Mechanisms of hyperglycemic response to chlorpromazine administered into lateral ventricle in rats-I: Possible role of dopaminergic nervous system".

The intestinal epithelium separates luminal contents from the interstitium. This function is primarily determined by the integrity of the epithelium and the tight junction that seals the paracellular space. These intestinal tight junctions are selectively permeable. This permeability can be increased physiologically in response to the presence of luminal nutrients. Permeability can also be increased pathologically by mucosal immune cells and cytokines, the enteric nervous system, and by pathogens. It is believed to be critical that the intestinal mucosa prevent potentially dangerous contents of the intestinal lumen, including the microorganisms that reside there from entering internal areas and the systemic circulation. There are several clinical conditions, both intestinal and systemic, that are associated with compromised intestinal barrier function.

A possible link between intestinal hyperpermeability and disease has been proposed. This has led to a sharp increase in the diagnosis of intestinal hyperpermeability, also known as "leaky gut syndrome." Diseases that have been correlated with intestinal hyperpermeability include diabetes, autism, fibromyalgia, inflammatory bowel disease (IBD), graft versus host disease (GVHD), HIV/AIDS, multiple organ dysfunction syndrome, irritable bowel syndrome (IBS), celiac disease, eczema, psoriasis, acute pancreatitis, Parkinson's disease, depression, chronic fatigue syndrome, asthma, multiple sclerosis, arthritis, ankylosing spondylitis, nonalcoholic fatty liver disease, alcoholic cirrhosis, environmental enteropathy, and kwashiorkor. It is believed that restoration of the intestinal barrier will improve or cure the underlying disease. Several drug targets that could potentially promote barrier restoration have been proposed, but none have proven safe and effective.

Thus, there remains a need for the development of safe and effective treatments or cures for intestinal hypersensitivity and numerous underlying diseases.

### SUMMARY

The present invention relates to a treatment regimen including: α-methyl-DL-tyrosine; melanotan II; 5,5-diphenylhydantoin; and N-[(2S,3R)-3-amino-2-hydroxy-4-phenylbutyryl]-L-leucine for use in the treatment of hyperglycemia in a human subject. Hyperglycemia is an underlying condition of intestinal hyperpermeability that may be treated by the claimed invention. The present disclosure provides methods, compositions, and kits for treating intestinal hyperpermeability in a subject in need thereof, including underlying diseases such as diabetes, autism, fibromyalgia, inflammatory bowel disease (IBD), graft versus host disease (GVHD), HIV/AIDS, multiple organ dysfunction syndrome, irritable bowel syndrome (IBS), celiac disease, eczema, psoriasis, acute pancreatitis, Parkinson's disease, depression, chronic fatigue syndrome, asthma, multiple sclerosis, arthritis, ankylosing spondylitis, nonalcoholic fatty liver disease, alcoholic cirrhosis, environmental enteropathy, or kwashiorkor. The disclosure provides methods comprising administering to a subject in need thereof an effective amount of a tyrosine hydroxylase inhibitor. The disclosure provides methods comprising administering to a subject in need thereof an effective amount of a tyrosine hydroxylase inhibitor and a p450 3A4 promoter.

The invention also provides pharmaceutical compositions and kits for use as defined in the claims.

### DETAILED DESCRIPTION OF ILLUSTRATIVE EMBODIMENTS

The present subject matter may be understood more readily by reference to the following detailed description which forms a part of this disclosure. It is to be understood that this invention is not limited to the specific products, methods, conditions or parameters described and/or shown herein, and that the terminology used herein is for the purpose of describing particular embodiments by way of example only and is not intended to be limiting of the claimed invention. The scope of the present invention, however, is defined by the appended claims. Any disclosure in the present description not protected by the appended claims does not form part of the present invention.

Unless otherwise defined herein, scientific and technical terms used in connection with the present application shall have the meanings that are commonly understood by those of ordinary skill in the art. Further, unless otherwise required by context, singular terms shall include pluralities and plural terms shall include the singular.

As employed above and throughout the disclosure, the following terms and abbreviations, unless otherwise indicated, shall be understood to have the following meanings.

In the present disclosure the singular forms "a," "an," and "the" include the plural reference, and reference to a particular numerical value includes at least that particular value, unless the context clearly indicates otherwise. Thus, for example, a reference to "a compound" is a reference to one or more of such compounds and equivalents thereof known to those skilled in the art, and so forth. The term "plurality", as used herein, means more than one. When a range of values is expressed, another embodiment incudes from the one particular and/or to the other particular value. Similarly, when values are expressed as approximations, by use of the antecedent "about," it is understood that the particular value forms another embodiment. All ranges are inclusive and combinable.

As used herein, the terms "component," "composition," "composition of compounds," "compound," "drug," "pharmacologically active agent," "active agent," "therapeutic," "therapy," "treatment," or "medicament" are used interchangeably herein to refer to a compound or compounds or composition of matter which, when administered to a subject (human or animal) induces a desired pharmacological and/or physiologic effect by local and/or systemic action.

As used herein, the terms "treatment" or "therapy" (as well as different forms thereof) include preventative (*e.g*., prophylactic), curative or palliative treatment. As used herein, the term "treating" includes alleviating or reducing at least one adverse or negative effect or symptom of a condition, disease or disorder. This condition, disease or disorder can be intestinal hyperpermeability.

As employed above and throughout the disclosure the term "effective amount" refers to an amount effective, at dosages, and for periods of time necessary, to achieve the desired result with respect to the treatment of the relevant disorder, condition, or side effect. It will be appreciated that the effective amount of components of the present invention will vary from patient to patient not only with respect to the particular compound, component or composition selected, the route of administration, and the ability of the components to elicit a desired result in the individual, but also with respect to factors such as the disease state or severity of the condition to be alleviated, hormone levels, age, sex, weight of the individual, the state of being of the patient, and the severity of the pathological condition being treated, concurrent medication or special diets then being followed by the particular patient, and other factors which those skilled in the art will recognize, with the appropriate dosage being at the discretion of the attending physician. Dosage regimes may be adjusted to provide improved therapeutic response. An effective amount is also one in which any toxic or detrimental effects of the components are outweighed by the therapeutically beneficial effects.

"Pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

"High blood glucose level" is used interchangeably with "hyperglycemia" herein and is defined as a fasting plasma blood glucose level of 126 mg/dl or greater on two separate occasions,

Within the present invention, the disclosed compounds may be prepared in the form of pharmaceutically acceptable salts. "Pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. These physiologically acceptable salts are prepared by methods known in the art, *e.g.,* by dissolving the free amine bases with an excess of the acid in aqueous alcohol, or neutralizing a free carboxylic acid with an alkali metal base such as a hydroxide, or with an amine.

Compounds described herein can be prepared in alternate forms. For example, many amino-containing compounds can be used or prepared as an acid addition salt. Often such salts improve isolation and handling properties of the compound. For example, depending on the reagents, reaction conditions and the like, compounds as described herein can be used or prepared, for example, as their hydrochloride or tosylate salts. Isomorphic crystalline forms, all chiral and racemic forms, N-oxide, hydrates, solvates, and acid salt hydrates, are also contemplated to be within the scope of the present invention.

Certain acidic or basic compounds of the present invention may exist as zwitterions. All forms of the compounds, including free acid, free base and zwitterions, are contemplated to be within the scope of the present invention. It is well known in the art that compounds containing both amino and carboxy groups often exist in equilibrium with their zwitterionic forms. Thus, any of the compounds described herein that contain, for example, both amino and carboxy groups, also include reference to their corresponding zwitterions.

The term "stereoisomers" refers to compounds that have identical chemical constitution, but differ as regards the arrangement of the atoms or groups in space. The term "enantiomers" refers to stereoisomers that are mirror images of each other that are non-superimposable.

The term "administering" means either directly administering a compound or composition of the present invention, or administering a prodrug, derivative or analog which will form an equivalent amount of the active compound or substance within the body.

The terms "subject," "individual," and "patient" are used interchangeably herein, and refer to an animal, for example a human, to whom treatment, including prophylactic treatment, with the pharmaceutical composition according to the present invention, is provided. The term "subject" as used herein refers to human and non-human animals. The terms "non-human animals" and "non-human mammals" are used interchangeably herein and include all vertebrates, *e.g.*, mammals, such as non-human primates, (particularly higher primates), sheep, dog, rodent, (*e.g.* mouse or rat), guinea pig, goat, pig, cat, rabbits, cows, horses and non-mammals such as reptiles, amphibians, chickens, and turkeys.

The term "inhibitor" as used herein includes compounds that inhibit the expression or activity of a protein, polypeptide or enzyme and does not necessarily mean complete inhibition of expression and/or activity. Rather, the inhibition includes inhibition of the expression and/or activity of a protein, polypeptide or enzyme to an extent, and for a time, sufficient to produce the desired effect.

The term "promoter" as used herein includes compounds that promote the expression or activity of a protein, polypeptide or enzyme and does not necessarily mean complete promotion of expression and/or activity. Rather, the promotion includes promotion of the expression and/or activity of a protein, polypeptide or enzyme to an extent, and for a time, sufficient to produce the desired effect.

While not intending to be bound by any particular mechanism of operation, it is believed that tyrosine hydroxylase inhibitors function by decreasing the amount of adrenaline secreted into the bloodstream.

Methods of treating intestinal hyperpermeability in a subject are described herein. Such methods can include administering to a subject in need thereof an effective amount of a tyrosine hydroxylase inhibitor. Other such methods include administering to a subject in need thereof an effective amount of tyrosine hydroxylase inhibitor and a p450 3A4 promoter. This tyrosine hydroxylase inhibitor and the p450 3A4 promoter can be administered simultaneously.

Administration of the tyrosine hydroxylase inhibitor or the tyrosine hydroxylase inhibitor and the p450 3A4 promoter can be through various routes, including orally, nasally subcutaneously, intravenously, intramuscularly, transdermally, vaginally, rectally or in any combination thereof. Transdermal administration can be effected using, for example, oleic acid, 1-methyl-2-pyrrolidone, dodecylnonaoxyethylene glycol monoether.

As described herein, the tyrosine hydroxylase inhibitor and the p450 3A4 promoter are administered during a cycle consisting of five to seven days of administering the tyrosine hydroxylase inhibitor and the p450 3A4 promoter, and one to two days of not administering the tyrosine hydroxylase inhibitor and the p450 3A4 promoter. At least six of said cycles of administration may be performed. 25 mg of the tyrosine hydroxylase inhibitor may be administered.

In the disclosure, the tyrosine hydroxylase inhibitor is a tyrosine derivative. The tyrosine derivative can be capable of existing in different isomeric forms, including stereoisomers and enantiomers. The tyrosine derivative can, for example, exist in both L-form or D-form. The tyrosine derivative can, for example, also exist in a racemic form. Tyrosine derivatives include one or more of methyl (2R)-2-amino-3-(2-chloro-4 hydroxyphenyl) propanoate, D-tyrosine ethyl ester hydrochloride, methyl (2R)-2- amino-3-(2,6-dichloro-3,4-dimethoxyphenyl) propanoate H-D-tyrosine(tBu)-allyl ester hydrochloride, methyl (2R)-2-amino-3-(3-chloro-4,5-dimethoxyphenyl) propanoate, methyl (2R)-2-amino-3-(2-chloro-3-hydroxy-4-methoxyphenyl) propanoate, methyl (2R)-2-amino-3-(4-[(2-chloro-6-fluorophenyl) methoxy] phenyl) propanoate, methyl (2R)-2- amino-3-(2-chloro-3,4-dimethoxyphenyl) propanoate, methyl (2R)-2-amino-3-(3-chloro-5-fluoro-4-hydroxyphenyl) propanoate, diethyl 2-(acetylamino)-2-(4-[(2-chloro-6-fluorobenzyl)oxy]benzyl malonate, methyl (2R)-2-amino-3-(3-chloro-4-methoxyphenyl) propanoate, methyl (2R)-2-amino-3-(3-chloro-4-hydroxy-5-methoxyphenyl) propanoate, methyl (2R)-2-amino-3-(2,6- dichloro-3-hydroxy-4-methoxyphenyl) propanoate, methyl (2R)-2-amino-3-(3-chloro-4-hydroxyphenyl) propanoate, H-DL-tyrosine methyl ester hydrochloride, H-3,5-diiodo-tyrosine methyl ester hydrochloride, H-D-3,5-diiodo-tyrosine methyl ester hydrochloride, H-D-tyrosine methyl ester hydrochloride, D-tyrosine methyl ester hydrochloride, D-tyrosine-methyl ester hydrochloride, methyl D-tyrosinate hydrochloride, H-D-tyrosine methyl ester•hydrochloride, D-tyrosine methyl ester hydrochloride, H-D-tyrosine methyl ester-hydrochloride, (2R)-2-amino-3-(4-hydroxyphenyl) propionic acid, (2R)-2-amino-3-(4-hydroxyphenyl) methyl ester hydrochloride, methyl (2R)-2-amino-3-(4-hydroxyphenyl) propanoate hydrochloride, methyl (2R)-2-azanyl-3-(4-hydroxyphenyl) propanoate hydrochloride, 3-chloro-L-tyrosine, 3-nitro-L-tyrosine, 3-nitro-L-tyrosine ethyl ester hydrochloride, DL-m-tyrosine, DL-o-tyrosine, Boc-tyrosine (3,5-I2)-OSu, Fmoc-tyrosine(3-NO2)-OH, α-methyl-L-tyrosine, α-methyl-D-tyrosine, and α-methyl-DL-tyrosine. A tyrosine derivative is α-methyl-L-tyrosine. Another tyrosine derivative is α-methyl-D-tyrosine. Another tyrosine derivative is α-methyl-DL-tyrosine in a racemic form.

As defined in the claims, 60 mg of the tyrosine derivative α-methyl-DL-tyrosine is administered orally and 0.25 mL of a 2 mg/mL suspension of the tyrosine derivative is administered subcutaneously.

p450 3A4 promoters include 5, 5-diphenylhydantoin, valproic acid and carbamazepine. In a suitable embodiment of the invention, the composition includes 5 mg to 25 mg of 5, 5-diphenylhydantoin. The subjects are human.

In some embodiments, the invention further comprises assessing progression of said subject, as defined in the claims. This assessing step can be performed before said administering step or after said administering step.

Representative conditions that can be treated with the present invention include hyperglycemia. Symptoms of the condition hyperglycemia can include: polyphagia, polydipsia, polyuria, blurred vision, fatigue (sleepiness), weight loss, poor wound healing (cuts, scrapes, etc.), dry mouth, dry or itchy skin, tingling in feet or heels, erectile dysfunction, recurrent infections, external ear infections (swimmer's ear), cardiac arrhythmia, stupor, coma, and seizures. Representative diseases that can be treated with methods described herein include diabetes, autism, fibromyalgia, inflammatory bowel disease (IBD), graft versus host disease (GVHD), HIV/AIDS, multiple organ dysfunction syndrome, irritable bowel syndrome (IBS), celiac disease, eczema, psoriasis, acute pancreatitis, Parkinson's disease, depression, chronic fatigue syndrome, asthma, multiple sclerosis, arthritis, ankylosing spondylitis, nonalcoholic fatty liver disease, alcoholic cirrhosis, environmental enteropathy, or kwashiorkor.

Administration of pharmaceutically active molecules such as inhibitor and/or promoters can be through various routes, including orally, nasally, subcutaneously, intravenously, intramuscularly, transdermally, vaginally, rectally or in any combination thereof. Transdermal administration can be effected using, for example, oleic acid, 1-methyl-2-pyrrolidone, dodecylnonaoxyethylene glycol monoether.

The tyrosine hydroxylase inhibitor of the treatment defined in the claims can be administered during a cycle consisting of five to seven days of administering the tyrosine hydroxylase inhibitor as defined in the claims, and one to two days of not administering the tyrosine hydroxylase inhibitor as defined in the claims. The tyrosine hydroxylase inhibitor as defined in the claims can be administered over the course of at least six said cycles. In one suitable embodiment of the invention, the tyrosine hydroxylase inhibitor as defined in the claims is administered daily. In another suitable embodiment of the invention, the tyrosine hydroxylase inhibitor as defined in the claims is administered multiple times per day.

Representative treatment according to the invention comprises administering to a subject in need thereof an effective amount of a tyrosine hydroxylase inhibitor or a tyrosine hydroxylase inhibitor and a p450 3A4 promoter as defined in the claims.

Suitable embodiments can include a pharmaceutical composition for use as defined in the claims.

Also provided herein are kits for use as defined in the claims.

The following examples are provided to supplement the prior disclosure and to provide a better understanding of the subject matter described herein. These examples should not be considered to limit the described subject matter.

### Example 1

Two-hundred patients were initially screened. Thirty subjects meeting the study criteria consented. Nine (9) subjects had high blood glucose levels (hyperglycemia) prior to consenting to the study.

A high blood glucose level (hyperglycemia) is defined as a fasting plasma blood glucose level of 126 mg/dl or greater on two separate occasions, The average patient age was sixty-two years old and the median patient age was sixty years old. Six of the patients were female and three of the patients were male. Five of the patients were fifty to sixty years old and four of the patients were over the age of sixty.

The patients in the study were administered a treatment regimen that included a tyrosine hydroxylase inhibitor (*i.e.,* α-methyl-DL tyrosine), a melanin promoter (*i.e.,* melanotan II), a p450 3A4 promoter (*i.e.,* 5, 5-diphenylhydantoin), and a leucine aminopeptidase inhibitor (*i.e.,* N-[(2S,3R)-3-amino-2-hydroxy-4-phenylbutyryl]-L-leucine). These compounds were administered on each of five days per week for a period of six weeks, with one or two days off between weekly cycles. Blood glucose level was monitored for all subjects biweekly. Blood glucose levels were determined by daily blood glucose tests followed-up with laboratory blood glucose tests every two weeks.

After approximately two to four weeks, all nine of the subjects had normal blood glucose levels defined as a fasting plasma blood glucose level of 125 mg/dl or lower on two separate occasions.

Overall, the above-noted treatment was well tolerated by the subjects, with no adverse events related to the treatment, and responses have been documented to the treatment 100%.

## Claims

1. A treatment regimen including:
• α-methyl-DL-tyrosine;
• melanotan II;
• 5,5-diphenylhydantoin; and
• N-[(2S,3R)-3-amino-2-hydroxy-4-phenylbutyryl]-L-leucine,
for use in the treatment of hyperglycemia in a human subject.

2. The treatment regimen for use according to claim 1, wherein the α-methyl-DL-tyrosine and the 5,5-diphenylhydantoin are administered simultaneously.

3. The treatment regimen for use according to claim 1 or 2, wherein the α-methyl-DL-tyrosine, or the α-methyl-DL-tyrosine and the 5,5-diphenylhydantoin are administered orally, subcutaneously, intravenously, transdermally, vaginally, rectally or in any combination thereof, optionally wherein the transdermal administration is performed in combination with oleic acid, 1-methyl-2-pyrrolidone, or dodecylnonaoxyethylene glycol monoether.

4. The treatment regimen for use according to any one of claims 1-3, wherein the α-methyl-DL-tyrosine, or the α-methyl-DL-tyrosine and the 5,5-diphenylhydantoin are administered during a cycle consisting of five to seven days of administering the α-methyl-DL-tyrosine or the α-methyl-DL-tyrosine and the 5,5-diphenylhydantoin, and one to two days of not administering the α-methyl-DL-tyrosine or the α-methyl-DL-tyrosine and the 5,5-diphenylhydantoin, optionally including at least six of said cycles.

5. The α-methyl-DL-tyrosine for use according to any one of claims 1-4; wherein 60 mg of the α-methyl-DL-tyrosine is administered orally and 0.25 mL of a 2 mg/mL suspension of the α-methyl-DL-tyrosine is administered subcutaneously.

6. The treatment regimen for use according to any one of claims 1-5, the treatment further comprising assessing progression of intestinal hyperpermeability in said subject, optionally wherein said assessing step is performed before said administering step, or wherein said assessing step is performed after said administering step.

7. The treatment regimen for use according to any one of claims 1-6, wherein the subject has a fasting plasma blood glucose level of 126 mg/dl or greater, as measured on two separate occasions, prior to treatment.

8. The treatment regimen for use according to any one of claims 1-7, provided as a pharmaceutical composition.

9. The treatment regimen for use according to any one of claims 1-8, wherein the α-methyl-DL-tyrosine and the 5,5-diphenylhydantoin are to be packaged in a kit.

## Patentansprüche

1. Behandlungsverfahren, das Folgendes einschließt:
• α-Methyl-DL-Tyrosin;
• Melanotan II;
• 5,5-Diphenylhydantoin und
• α-N-[(2S,3R)-3-Amino-2-hydroxy-4-phenylbutyryl]-L-Leucin,
zur Verwendung bei der Behandlung von Hyperglykämie bei einem Menschen.

2. Behandlungsverfahren zur Verwendung nach Anspruch 1, wobei das α-Methyl-DL-Tyrosin und das 5,5-Diphenylhydantoin gleichzeitig verabreicht werden.

3. Behandlungsverfahren zur Verwendung nach Anspruch 1 oder 2, wobei das α-Methyl-DL-Tyrosin bzw. das α-Methyl-DL-Tyrosin und das 5,5-Diphenylhydantoin oral, subkutan, intravenös, transdermal, vaginal, rektal oder in einer beliebigen Kombination davon verabreicht werden, wobei die transdermale Verabreichung optional in Kombination mit Oleinsäure, 1-Methyl-2-pyrrolidon oder Dodecylnonaoxyethylenglykolmonoether durchgeführt wird.

4. Behandlungsverfahren zur Verwendung nach einem der Ansprüche 1 bis 3, wobei das α-Methyl-DL-Tyrosin bzw. das α-Methyl-DL-Tyrosin und das 5,5-Diphenylhydantoin während eines Zyklus verabreicht werden, der aus fünf bis sieben Tagen der Verabreichung des α-Methyl-DL-Tyrosins bzw. des α-Methyl-DL-Tyrosins und des 5,5-Diphenylhydantoins und einem bis zwei Tagen ohne Verabreichung des α-Methyl-DL-Tyrosins bzw. des α-Methyl-DL-Tyrosins und des 5,5-Diphenylhydantoins besteht, wobei optional mindestens sechs dieser Zyklen eingeschlossen sind.

5. α-Methyl-DL-Tyrosin zur Verwendung nach einem der Ansprüche 1 bis 4, wobei 60 mg des α-Methyl-DL-Tyrosins oral verabreicht werden und 0,25 ml einer 2 mg/ml-Suspension des α-Methyl-DL-Tyrosins subkutan verabreicht werden.

6. Behandlungsverfahren zur Verwendung nach einem der Ansprüche 1 bis 5, wobei die Behandlung ferner die Beurteilung des Verlaufs der intestinalen Hyperpermeabilität bei dem Patienten umfasst, wobei optional der Beurteilungsschritt vor dem Verabreichungsschritt durchgeführt wird bzw. der Beurteilungsschritt nach dem Verabreichungsschritt durchgeführt wird.

7. Behandlungsverfahren zur Verwendung nach einem der Ansprüche 1 bis 6, wobei der Patient einen Nüchternplasmablutzuckerspiegel von 126 mg/dl oder mehr aufweist, der zu zwei verschiedenen Zeitpunkten vor der Behandlung gemessen wurde.

8. Behandlungsverfahren zur Verwendung nach einem der Ansprüche 1 bis 7, bereitgestellt als eine pharmazeutische Zusammensetzung.

9. Behandlungsverfahren zur Verwendung nach einem der Ansprüche 1 bis 8, wobei das α-Methyl-DL-Tyrosin und das 5,5-Diphenylhydantoin in einem Packungsset zu verpacken sind.

## Revendications

1. Régime de traitement comportant :
• de la α-méthyl-DL-tyrosine ;
• du mélanotan II ;
• de la 5,5-diphénylhydantoïne ; et
• de la N-[(2S,3R)-3-amino-2-hydroxy-4-phénylbutyryl]-L-leucine,
pour une utilisation dans le traitement de l'hyperglycémie chez un sujet humain.

2. Régime de traitement pour une utilisation selon la revendication 1, la α-méthyl-DL-tyrosine et la 5,5-diphénylhydantoïne étant administrées simultanément.

3. Régime de traitement pour une utilisation selon la revendication 1 ou 2, la α-méthyl-DL-tyrosine, ou la α-méthyl-DL-tyrosine et la 5,5-diphénylhydantoïne étant administrée(s) par voie orale, sous-cutanée, intraveineuse, transdermique, vaginale, rectale ou en une quelconque combinaison correspondante, éventuellement, l'administration transdermique étant réalisée en combinaison avec de l'acide oléique, de la 1-méthyl-2-pyrrolidone, ou du monoéther de dodécylnonaoxyéthylène glycol.

4. Régime de traitement pour une utilisation selon l'une quelconque des revendications 1 à 3, la α-méthyl-DL-tyrosine, ou la α-méthyl-DL-tyrosine et la 5,5-diphénylhydantoïne étant administrée(s) pendant un cycle constitué de cinq à sept jours d'administration de la α-méthyl-DL-tyrosine, ou de la α-méthyl-DL-tyrosine et de la 5,5-diphénylhydantoïne, et d'un à deux jours de non administration de la α-méthyl-DL-tyrosine, ou de la α-méthyl-DL-tyrosine et de la 5,5-diphénylhydantoïne, éventuellement comportant au moins six desdits cycles.

5. α-méthyl-DL-tyrosine pour une utilisation selon l'une quelconque des revendications 1 à 4 ; 60 mg de la α-méthyl-DL-tyrosine étant administrés par voie orale et 0,25 mL d'une suspension à 2 mg/mL de la α-méthyl-DL-tyrosine étant administré par voie sous-cutanée.

6. Régime de traitement pour une utilisation selon l'une quelconque des revendications 1 à 5, le traitement comprenant en outre l'évaluation de la progression de l'hyperperméabilité intestinale chez ledit sujet, éventuellement ladite étape d'évaluation étant réalisée avant ladite étape d'administration, ou ladite étape d'évaluation étant réalisée après ladite étape d'administration.

7. Régime de traitement pour une utilisation selon l'une quelconque des revendications 1 à 6, le sujet possédant une glycémie plasmatique à jeun de 126 mg/dl ou plus, telle que mesurée en deux occasions distinctes, avant le traitement.

8. Régime de traitement pour une utilisation selon l'une quelconque des revendications 1 à 7, fournie en tant que composition pharmaceutique.

9. Régime de traitement pour une utilisation selon l'une quelconque des revendications 1 à 8, la α-méthyl-DL-tyrosine et la 5,5-diphénylhydantoïne devant être conditionnées dans un kit.
